# FASCICULE DE BREVET EUROPEEN

(11) **EP 0 904 769 B1**
(45) Date de publication et mention de la délivrance du brevet: **25.09.2002**
(21) Numéro de dépôt: 98401798.8
(22) Date de dépôt: 16.07.1998
(51) Int. Cl.: A61K 7/13

(54) **Compositions de teinture des fibres kératiniques contenat des dérivés d'indazoles amines et procédé de teinture**
Zusammensetzung zur fäbung von Keratinischen fasern enthaltend Indazol-Amin-Derivate und Färbeverfahren
Keratin fibre dye compositions containing Indazole amine derivatives and dyeing method

(30) Priorité: 25.08.1997 FR 9710618
(43) Date de publication de la demande: 31.03.1999
(73) Titulaire: L'OREAL, 75008 Paris (FR)
(72) Inventeur: Vandenbossche, Jean-Jacques, 40400 Begaar (FR); Lagrange, Alain, 77700 Coupvray (FR)
(74) Mandataire: Goulard, Sophie

(56) Documents cités:
- DE-A- 2 719 179
- FR-A- 2 315 906
- FR-A- 2 352 541
- US-A- 4 013 404

## Description

L'invention a pour objet une composition pour la teinture d'oxydation des fibres kératiniques contenant au moins un dérivé d'indazole amine à titre de coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet différemment sensibilisée (i.e. abîmée) entre sa pointe et sa racine.

L'utilisation de composés de type indazole est connue dans le domaine de la coloration des cheveux. La demande de brevet DE-A-1 492 166 décrit la polycondensation par oxydation de tels composés. La demande de brevet DE-A-2 623 564 décrit l'association d'hydroxy indazoles avec des tétraamino pyrimidines. La demande de brevet DE-A-2 719 179 décrit l'association de diamino indazoles avec des tétraamino pyrimidines. Le brevet US 4 013 404 décrit certains amino indazoles ainsi que leur utilisation à titre de précurseurs de colorants d'oxydation.

La demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des dérivés d'indazole amine particuliers.

Cette découverte est à la base de la présente invention.

L'invention a donc pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un dérivé d'indazole amine choisi parmi :
   ■ les composés de formule (II), et leurs sels d'addition avec un acide : 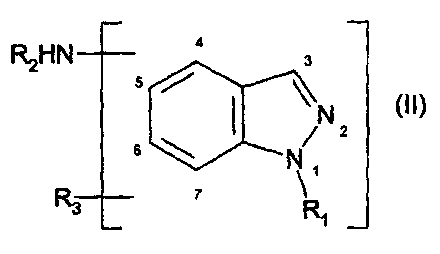 dans laquelle :
      - R₁ désigne un atome d'hydrogène ou un radical choisi parmi les radicaux alkyle en C₁-C₄, hydroxyalkyle en C₁-C₄, aminoalkyle en C₁-C₄, monoalkyl (C₁-C₄)aminoalkyle en C₁-C₄, dialkyl(C₁-C₄)aminoalkyle en C₁-C₄, alcoxy (C₁-C₄)carbonyle, alcoxy(C₁-C₄)carbonylalkyle en C₁-C₄, acyle en C₁-C₄, alcoxy(C₁-C₄ )alkyle en C₁-C₄, cyanoalkyle en C₁-C₄, allyle, alkyl (C₁-C₄)thiocarbonylalkyle en C₁-C₄ ;
      - R₂ désigne un atome d'hydrogène ou un radical choisi parmi les radicaux alkyle en C₁-C₄, hydroxyalkyle en C₁-C₄, aminoalkyle en C₁-C₄, monoalkyl (C₁-C₄)aminoalkyle en C₁-C₄, dialkyl(C₁-C₄)aminoalkyle en C₁-C₄, carboxyalkyle en C₁-C₄, alcoxy(C₁-C₄)alkyle en C₁-C₄, ω-hydroxyalcoxy (C₁-C₄)alkyle en C₁-C₄ ;
      - R₃ désigne un atome d'hydrogène, un atome de brome, de chlore, de fluor, ou un radical choisi parmi les radicaux alkyle en C₁-C₄, hydroxyalkyle en C₁-C₄, aminoalkyle en C₁-C₄, alcoxy en C₁-C₄, alcoxy(C₁-C₄)alkyle en C₁-C₄, monoalkyl(C₁-C₄)aminoalkyle en C₁-C₄, dialkyl(C₁-C₄)aminoalkyle en C₁-C₄, perfluoroalkyle en C₁-C₄, cyanoalkyle en C₁-C₄, ou carboxyalkyle en C₁-C₄ ;
      étant entendu que :
      - lorsque R₁ et R₂ représentent simultanément un atome d'hydrogène, alors R₃ est différent d'un radical alcoxy en C₁ ;
   ■ et les composés de formule (III), et leurs sels d'addition avec un acide : 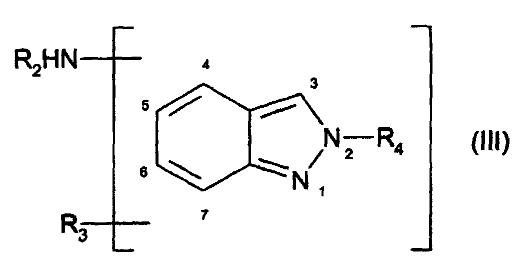 dans laquelle :
      - R₄ désigne un radical choisi parmi les radicaux alkyle en C₁-C₄, hydroxyalkyle en C₁-C₄, aminoalkyle en C₁-C₄, monoalkyl(C₁-C₄)aminoalkyle en C₁-C₄, dialkyl(C₁-C₄)aminoalkyle en C₁-C₄, alcoxy(C₁-C₄)carbonyle, acyle en C₁-C₄, alkylaryle en C₇-C₉, un radical imidino (-C(NH₂)=NH) ;
      - R₂ désigne un atome d'hydrogène ou un radical choisi parmi les radicaux alkyle en C₁-C₄, hydroxyalkyle en C₁-C₄, aminoalkyle en C₁-C₄, monoalkyl(C₁-C₄)aminoalkyle en C₁-C₄, dialkyl(C₁-C₄)aminoalkyle en C₁-C₄, carboxyalkyle en C₁-C₄, alcoxy(C₁-C₄)alkyle en C₁-C₄, ω-hydroxyalcoxy (C₁-C₄)alkyle en C₁-C₄ ;
      - R₃ désigne un atome d'hydrogène, un atome de brome, de chlore, de fluor, ou un radical choisi parmi les radicaux alkyle en C₁-C₄, hydroxyalkyle en C₁-C₄, aminoalkyle en C₁-C₄, alcoxy en C₁-C₄, alcoxy(C₁-C₄)alkyle en C₁-C₄, monoalkyl(C₁-C₄)aminoalkyle en C₁-C₄, dialkyl(C₁-C₄)aminoalkyle en C₁-C₄, perfluoroalkyle en C₁-C₄, cyanoalkyle en C₁-C₄, carboxyalkyle en C₁-C₄, où un groupement amino ;
- et au moins une base d'oxydation.

Dans les formules (II) et (III) ci-dessus, les groupements alkyle en C₁-C₄ et alcoxy en C₁-C₄ peuvent être linéaires ou ramifiés.

Les composés de formules (II) et (III) ci-dessus sont des composés connus, dont les modes de préparation sont décrits dans les ouvrages suivants :
- Barton D., Ollis W; D. , "Comprehensive organic chemistry", vol 4, page 400-410, édition Pergamon Press;
- Fusco R., "The chemistry of heterocyclic compounds, Pyrazoles, Pyrazolines, Pyrazolidines, Indazoles and condensed rings", 1967, édité par R. H. Wiley N. Y.
- Katritsky A., "Comprehensive heterocyclic chemistry", édition Elsevier.

Les colorations obtenues avec la composition de teinture conforme à l'invention, sont de nuances variées, puissantes, peu sélectives et présentent d'excellentes propriétés de résistance à la fois vis-à-vis des agents atmosphériques tels que la lumière et les intempéries et vis-à-vis de la transpiration et des différents traitements que peuvent subir les cheveux (shampooings, déformations permanentes).

Parmi les dérivés d'indazoles amines de formules (II) ou (III), utilisables à titre de coupleurs dans les compositions tinctoriales conformes à l'invention, on peut notamment citer :
- le 4-amino 1H-indazole,
- le 5-amino 1H-indazole,
- le 6-amino 1H-indazole,
- le 7-amino 1H-indazole,
- le 4-(2'-amino-éthyl)-amino 1H-indazole,
- le 5-(2'-amino-éthyl)-amino 1H-indazole,
- le 4-amino 6-méthyl 1H-indazole,
- le 3-chloro 4-amino 1H-indazole,
- le 4-amino 7-méthyl 1H-indazole,
- le 4-amino 5-méthyl 1H-indazole,
- le 4-(3'-amino-propyl)-amino 1H-indazole,
- le 3-fluoro 5-amino 1H-indazole,
- le 4-méthyl 5-amino 1H-indazole,
- le 3-méthyl 5-amino 1H-indazole,
- le 3-[(2'-N,N-diéthyl)-amino propyl]-amino 5-amino 1H-indazole,
- le 5-(2'-amino éthyl)-amino 1H-indazole,
- le 5-amino 6-méthyl 1H-indazole,
- le 3-trifluorométhyl 5-amino 1H-indazole,
- l'acide (5-amino 1H-indazol-3-yl) acétique,
- le 3-chloro 5-amino 1H-indazole,
- le 6-amino 7-chloro 1H-indazole,
- le 5-amino 6-chloro 1H-indazole,
- le 6-amino 7-bromo 1H-indazole,
- l'ester éthylique de l'acide (6-amino 3-chloro 1H-indazol-1-yl) acétique,
- le 1-méthoxyméthyl 6-amino 1H-indazole,
- le 1-allyl 6-amino 1H-indazole,
- le 6-amino 7-chloro 1H-indazole,
- racide (1H-indazol-6-yl amino) acétique,
- le 6-N-(2'-hydroxy-éthyl)-amino 1H-indazole,
- le 6-(N-propyl)-amino 1H-indazole,
- le 1-(N',N'-diéthyl amino)-éthyl 6-amino 1H-indazole,
- le 5-chloro 6-amino 1H-indazole,
- le 1-(2'-cyano)-éthyl 6-amino 1H-indazole,
- le 3-(6-amino 1H-indazol-1-yl) thiopropionamide,
- le 6-(N-éthyl)-amino 1H-indazole,
- le 3-méthyl 6-amino 1H-indazole,
- le 5-méthyl 6-amino 1H-indazole,
- le 3-bromo 6-amino 1H-indazole,
- le 3-chloro 6-amino 1H-indazole,
- le 6-amino 7-méthyl 1H-indazole,
- le 4-chloro 6-amino 1H-indazole,
- le 5-méthyl 7-(2'-méthoxy éthyl)-amino 1H-indazole,
- le 5-méthyl 7-(2'-hydroxy éthyl)-amino 1H-indazole,
- le 6-méthyl 7-[2'-(2"-hydroxy)-éthoxy éthyl]-amino 1H-indazole,
- le 4-(2'-N,N-dipropyl)-éthyl 7-amino 1H-indazole,
- le 4-(2'-amino)-éthyl 7-amino 1H-indazole,
- le 4-cyanométhyl 7-amino 1H-indazole,
- le 2-(2'-N,N-diméthyl)-éthyl 7-amino 1H-indazole,
- le 3-méthyl 7-amino 1H-indazole,
- le 4-chloro 7-amino 1H-indazole,
- le 7-[N-(2'-amino)-éthyl]-amino 1H-indazole,
- le 4-chloro 5-méthyl 7-amino 1H-indazole,
- le 5-chloro 7-amino 1H-indazole,
- le 5-méthyl 7-amino 1H-indazole,
- le 6-méthyl 7-amino 1H-indazole,
- le 1-méthyl 4-amino 1H-indazole,
- le 1-méthyl 5-amino 1H-indazole,
- le 1-méthyl 6-amino 1H-indazole,
- le 1,3-diméthyl 6-amino 1H-indazole,
- le 1-méthyl 7-amino 1H-indazole,
- le 1,5-diméthyl 7-amino 1H-indazole,
- le 1-méthyl 3-bromo 7-(N-méthyl)-amino 1H-indazole,
- le 1-méthyl 3-chloro 7-(N-méthyl)-amino 1H-indazole,
- le 1-méthyl 5-chloro 7-amino 1H-indazole,
- le 1-méthyl 7-(N-méthyl)-amino 1H-indazole,
- le 1-méthyl 6-chloro 7-amino 1H-indazole,
- le 1-éthyl 7-amino 1H-indazole,
- le 2-méthyl 3-amino 5-trifluorométhyl 2H-indazole,
- le 2-méthyl 3- (N-méthyl) amino 2H-indazole,
- le 2-méthyl 4-amino 2H-indazole,
- le 2,6-diméthyl 4-amino 2H-indazole,
- le 2-méthyl 5-amino 2H-indazole,
- le 2,3-diméthyl 5-amino 2H-indazole,
- le 2-méthyl 6-amino 2H-indazole,
- le 2-méthyl 7-amino 2H-indazole,
- le 2-éthyl 5-amino 2H-indazole,
- le 2-éthyl 6-amino 2H-indazole,
- le 2-benzyl 4-amino 2H-indazole,
- le 2-(2'-N,N-diméthyl)-aminoéthyl 6-amino 2H-indazole,
- le 2-(2'-N,N-diéthyl)-aminoéthyl 6-amino 2H-indazole,
- le 2-imidino 5-amino 2H-indazole,
- le 2-imidino 6-amino 2H-indazole,
- le 2-imidino 7-amino 2H-indazole,
et leurs sels d'addition avec un acide.

Le ou les dérivés d'indazoles amines de formules (II) et/ou (III) et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les para-aminophénols, les ortho-aminophénols, les bases hétérocycliques, et leurs sels d'addition avec un acide.

Parmi les paraphénylènediamines utilisables à titre de bases d'oxydation dans la composition tinctoriale conforme à l'invention, on peut notamment citer les composés répondant à la formule (IV) suivante, et leurs sels d'addition avec un acide : 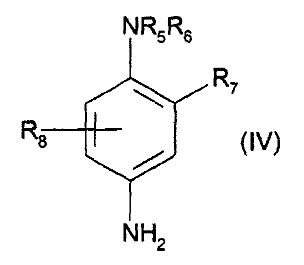 dans laquelle :
- R₅ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄);
- R₆ représente un atome d'hydrogène, un radical alkyle en C₁-C₄, monohydroxyalkyle en C₁-C₄ ou polyhydroxyalkyle en C₂-C₄ ;
- R₇ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄ ;
- R₈ représente un atome d'hydrogène ou un radical alkyle en C₁-C₄.

Dans la formule (IV) ci-dessus, et lorsque R₇ est différent d'un atome d'hydrogène, alors R₅ et R₆ représentent de préférence un atome d'hydrogène et R₇ est de préférence identique à R₈, et lorsque R₇ représente un atome d'halogène, alors R₅, R₆ et R₈ représentent de préférence un atome d'hydrogène.

Parmi les paraphénylènediamines de formule (IV) ci-dessus, on peut plus particulièrement citer la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxyda dans la composition tinctoriale conforme à l'invention, on peut notamment les composés répondant à la formule (V) suivante, et leurs sels d'addition a un acide 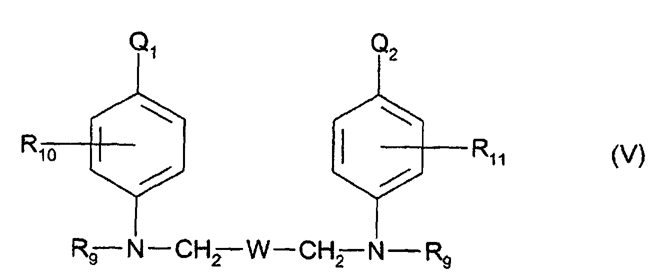 dans laquelle :
- Q₁ et Q₂, identiques ou différents, représentent un radical hydroxyle ou NHR₁₂ dans lequel R₁₂ représente un atome d'hydrogène ou un radical alkyle en C₁-C₄,
- R₉ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
- R₁₀ et R₁₁, identiques ou différents, représentent un atome d'hydrogène ou d'halogène ou un radical alkyle en C₁-C₄,
- W représente un radical pris dans le groupe constitué par les radicaux suivants :
   -(CH₂)ₙ ; ―(CH₂)ₘ-O-(CH₂)ₘ― ; ―(CH₂)ₘ-CHOH-(CH₂)ₘ― et 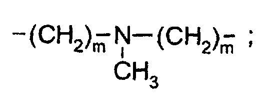
dans lesquels n est un nombre entier compris entre 0 et 8 inclusivement et m est un nombre entier compris entre 0 et 4 inclusivement.

Parmi les bis-phénylalkylènediamines de formule (V) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les bis-phénylalkylènediamines de formule (V) ci-dessus, le N,N'-bis-(β-hydroxyéthyl) N,N'-bis-(4'-aminophényl) 1,3-diamino 2-propanol et l'un de ses sels d'addition avec un acide sont particulièrement préférés.

Parmi les para-aminophénols utilisables à titre de bases d'oxydation dans la composition tinctoriale conforme à l'invention, on peut notamment citer les composés répondant à la formule (VI) suivante, et leurs sels d'addition avec un acide : 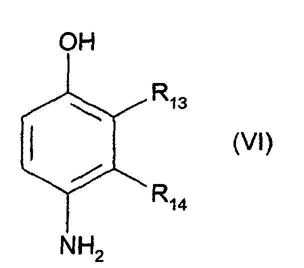 dans laquelle :
- R₁₃ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄ ;
- R₁₄ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄) ;
étant entendu qu'au moins un des radicaux R₁₃ ou R₁₄ représente un atome d'hydrogène.

Parmi les para-aminophénols de formule (VI) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, leurs sels d'addition et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon l'invention, la ou les bases d'oxydation représentent de préférence de 0,0005 à12 % en poids environ du poids total de la composition tinctoriale, et encore plus préférentiellement de 0,005 à 6 % en poids environ de ce poids.

La composition tinctoriale conforme à l'invention peut également renfermer un ou plusieurs coupleurs additionnels différents des dérivés d'indazoles amines de formules (II) et (III) et/ou un ou plusieurs colorants directs de façon à faire varier ou enrichir en reflets les nuances obtenues.

Les coupleurs additionnels utilisables dans la composition conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-β-hydroxyéthyloxy benzène, le 2-amino 4-N-(β-hydroxyéthyl)amino 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents, ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide du ou des dérivés d'indazole amine de formule (II) et (III), et/ou de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol, les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VII) suivante : 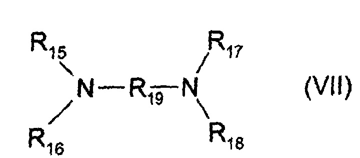 dans laquelle R₁₉ est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des dérivés d'indazoles amines de formules (II) ou (III) ci-dessus, à titre de coupleur, en association avec au moins une base d'oxydation, pour la teinture d'oxydation des fibres kératiniques et en particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale telle que définie ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides et les enzymes. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture à plusieurs compartiments ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES 1 A 20 :

On a préparé les compositions tinctoriales suivantes :
- Coupleur 0,003 mole
- Base 0,003 mole
- Alcool absolu 30 g
- Solution aqueuse de métabisulfite de sodium à 35 % 1,3 g
- Solution aqueuse d'ammoniaque à 20 % qs pH 10
- Eau qsp 100 g

Ces compositions tinctoriales ont été préparées pour les coupleurs conformes à l'invention suivants :
C1 : 2-méthyl 4-amino 2H-indazole, 2 HCl
C2 : 2-méthyl 5-amino 2H-indazole, 2 HCl
C3 : 2-méthyl 6-amino 2H-indazole, 2 HCl
C4 : 2-méthyl 7-amino 2H-indazole, HCl
C5 : 1-méthyl 4-amino 1H-indazole, 2 HCl
C6 : 1-méthyl 5-amino 1H-indazole, HCl, H₂O
C7 : 1-méthyl 6-amino 1H-indazole
C8 : 1-méthyl 7-amino 1H-indazole, HCl, H₂O
C9 : 4-amino 1H-indazole, 2 HCl
C10 : 7-amino 1H-indazole, 2 HCl

Pour chaque coupleur, on a préparé deux compositions tinctoriales avec chacune une base d'oxydation différente : la paraphénylènediamine (PPD) ou le para-aminophénol (PAP).

Au moment de l'emploi, chaque composition tinctoriale a été mélangée avec un poids égal de peroxyde d'hydrogène à 20 volumes (6 % en poids).

Chaque mélange obtenu a été appliqué pendant 30 minutes, sur des mèches de cheveux gris naturels à 90 % de blancs, à raison de 28 g pour 3 g de cheveux. Après rinçage, lavage avec un shampooing standard et séchage, les mèches ont été teintées dans les nuances figurant dans le tableau I ci-dessous :

**Tableau I**

| **Exemple** | **Coupleur** | **Base** | **Nuance obtenue** |
|---|---|---|---|
| 1 | C1 | PPD | Violine |
| 2 | C1 | PAP | Cuivré rosé |
| 3 | C2 | PPD | Aubergine à reflet doré |
| 4 | C2 | PAP | Blond cuivré |
| 5 | C3 | PPD | Gris foncé à reflet aubergine |
| 6 | C3 | PAP | Miel |
| 7 | C4 | PPD | Pourpre bleu brillant |
| 8 | C4 | PAP | Bois de rose très intense |
| 9 | C5 | PPD | Châtain violine |
| 10 | C5 | PAP | Bois de rose doré |
| 11 | C6 | PPD | Châtaigne |
| 12 | C6 | PAP | Blond foncé cuivré |
| 13 | C7 | PPD | Gris cendré à reflet violine |
| 14 | C7 | PAP | Blond légèrement cuivré |
| 15 | C8 | PPD | Bleu gris violacé |
| 16 | C8 | PAP | Blond doré légèrement cuivré |
| 17 | C9 | PPD | Châtain violine |
| 18 | C9 | PAP | Bois de rose |
| 19 | C10 | PPD | Pourpre bleu intense |
| 20 | C10 | PAP | Noisette |

## Revendications

1. Composition pour la teinture des fibres kératiniques et en particulier des fibres kératiniques humaines telles que les cheveux, **caractérisée par le fait qu'**elle comprend, dans un milieu approprié pour la teinture :
- à titre de coupleur, au moins un dérivé d'indazole amine choisi parmi :
■ les composés de formule (II), et leurs sels d'addition avec un acide : 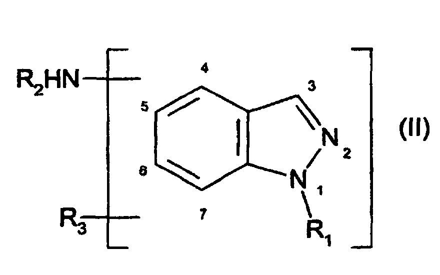 dans laquelle:
- R₁ désigne un atome d'hydrogène ou un radical choisi parmi les radicaux alkyle en C₁-C₄, hydroxyalkyle en C₁-C₄, aminoalkyle en C₁-C₄, monoalkyl (C₁-C₄)aminoalkyle en C₁-C₄, dialkyl(C₁-C₄)aminoalkyle en C₁-C₄, alcoxy (C₁-C₄)carbonyle, alcoxy(C₁-C₄)carbonylalkyle en C₁-C₄, acyle en C₁-C₄, alcoxy(C₁-C₄)alkyle en C₁-C₄, cyanoalkyle en C₁-C₄, allyle, alkyl (C₁-C₄)thiocarbonylalkyle en C₁-C₄ ;
- R₂ désigne un atome d'hydrogène ou un radical choisi parmi les radicaux alkyle en C₁-C₄, hydroxyalkyle en C₁-C₄, aminoalkyle en C₁-C₄, monoalkyl (C₁-C₄)aminoalkyle en C₁-C₄, dialkyl(C₁-C₄)aminoalkyle en C₁-C₄, carboxyalkyle en C₁-C₄, alcoxy(C₁-C₄)alkyle en C₁-C₄, ω-hydroxyalcoxy (C₁-C₄)alkyle en C₁-C₄ ;
- R₃ désigne un atome d'hydrogène, un atome de brome, de chlore, de fluor, ou un radical choisi parmi les radicaux alkyle en C₁-C₄, hydroxyalkyle en C₁-C₄, aminoalkyle en C₁-C₄, alcoxy en C₁-C₄, alcoxy(C₁-C₄)alkyle en C₁-C₄, monoalkyl(C₁-C₄)aminoalkyle en C₁-C₄, dialkyl(C₁-C₄)aminoalkyle en C₁-C₄, perfluoroalkyle en C₁-C₄, cyanoalkyle en C₁-C₄, ou carboxyalkyle en C₁-C₄ ;
étant entendu que :
- lorsque R₁ et R₂ représentent simultanément un atome d'hydrogène, alors R₃ est différent d'un radical alcoxy en C₁ ;
■ et les composés de formule (III), et leurs sels d'addition avec un acide : 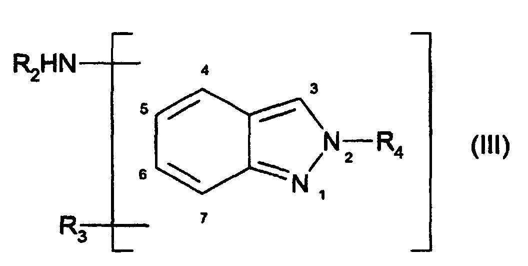 dans laquelle :
- R₄ désigne un radical choisi parmi les radicaux alkyle en C₁-C₄, hydroxyalkyle en C₁-C₄, aminoalkyle en C₁-C₄, monoalkyl(C₁-C₄)aminoalkyle en C₁-C₄, dialkyl(C₁-C₄)aminoalkyle en C₁-C₄, alcoxy(C₁-C₄)carbonyle, acyle en C₁-C₄, alkylaryle en C₇-C₉, un radical imidino (-C(NH₂)=NH) ;
- R₂ désigne un atome d'hydrogène ou un radical choisi parmi les radicaux alkyle en C₁-C₄, hydroxyalkyle en C₁-C₄, aminoalkyle en C₁-C₄, monoalkyl(C₁-C₄)aminoalkyle en C₁-C₄, dialkyl(C₁-C₄)aminoalkyle en C₁-C₄, carboxyalkyle en C₁-C₄, alcoxy(C₁-C₄)alkyle en C₁-C₄, ω-hydroxyalcoxy (C₁-C₄)alkyle en C₁-C₄ ;
- R₃ désigne un atome d'hydrogène, un atome de brome, de chlore, de fluor, ou un radical choisi parmi les radicaux alkyle en C₁-C₄, hydroxyalkyle en C₁-C₄, aminoalkyle en C₁-C₄, alcoxy en C₁-C₄, alcoxy(C₁-C₄)alkyle en C₁-C₄, monoalkyl(C₁-C₄)aminoalkyle en C₁-C₄, dialkyl(C₁-C₄)aminoalkyle en C₁-C₄, perfluoroalkyle en C₁-C₄, cyanoalkyle en C₁-C₄, carboxyalkyle en C₁-C₄, ou un groupement amino ;
- et au moins une base d'oxydation.

2. Composition selon la revendication 1, **caractérisée par le fait que** les dérivés d'indazoles amines de formules (II) ou (III) sont choisis parmi :
- le 4-amino 1H-indazole,
- le 5-amino 1H-indazole,
- le 6-amino 1H-indazole,
- le 7-amino 1H-indazole,
- le 4-(2'-amino-éthyl)-amino 1H-indazole,
- le 5-(2'-amino-éthyl)-amino 1H-indazole,
- le 4-amino 6-méthyl 1H-indazole,
- le 3-chloro 4-amino 1H-indazole,
- le 4-amino 7-méthyl 1H-indazole,
- le 4-amino 5-méthyl 1H-indazole,
- le 4-(3'-amino-propyl)-amino 1H-indazole,
- le 3-fluoro 5-amino 1H-indazole,
- le 4-méthyl 5-amino 1H-indazole,
- le 3-méthyl 5-amino 1H-indazole,
- le 3-[(2'-N,N-diéthyl)-amino propyfl-amino 5-amino 1H-indazole,
- le 5-(2'-amino éthyl)-amino 1H-indazole,
- le 5-amino 6-méthyl 1H-indazole,
- le 3-trifluorométhyl 5-amino 1H-indazole,
- l'acide (5-amino 1H-indazol-3-yl) acétique,
- le 3-chloro 5-amino 1H-indazole,
- le 6-amino 7-chloro 1H-indazole,
- le 5-amino 6-chloro 1H-indazole,
- le 6-amino 7-bromo 1H-indazole,
- l'ester éthylique de l'acide (6-amino 3-chloro 1H-indazol-1-yl) acétique,
- le 1-méthoxyméthyl 6-amino 1H-indazole,
- le 1-allyl 6-amino 1H-indazole,
- le 6-amino 7-chloro 1H-indazole,
- l'acide (1H-indazol-6-yl amino) acétique,
- le 6-N-(2'-hydroxy-éthyl)-amino 1H-indazole,
- le 6-(N-propyl)-amino 1H-indazole,
- le 1-(N',N'-diéthyl amino)-éthyl 6-amino 1H-indazole,
- le 5-chloro 6-amino 1H-indazole,
- le 1-(2'-cyano)-éthyl 6-amino 1H-indazole,
- le 3-(6-amino 1H-indazol-1-yl) thiopropionamide,
- le 6-(N-éthyl)-amino 1H-indazole,
- le 3-méthyl 6-amino 1H-indazole,
- le 5-méthyl 6-amino 1H-indazole,
- le 3-bromo 6-amino 1H-indazole,
- le 3-chloro 6-amino 1H-indazole,
- le 6-amino 7-méthyl 1H-indazole,
- le 4-chloro 6-amino 1H-indazole,
- le 5-méthyl 7-(2'-méthoxy éthyl)-amino 1H-indazole,
- le 5-méthyl 7-(2'-hydroxy éthyl)-amino 1H-indazole,
- le 6-méthyl 7-[2'-(2"-hydroxy)-éthoxy éthyl]-amino 1H-indazole,
- le 4-(2'-N,N-dipropyl)-éthyl 7-amino 1H-indazole,
- le 4-(2'-amino)-éthyl 7-amino 1H-indazole,
- le 4-cyanométhyl 7-amino 1H-indazole,
- le 2-(2'-N,N-diméthyl)-éthyl 7-amino 1H-indazole,
- le 3-méthyl 7-amino 1H-indazole,
- le 4-chloro 7-amino 1H-indazole,
- le 7-[N-(2'-amino)-éthyl]-amino 1H-indazole,
- le 4-chloro 5-méthyl 7-amino 1H-indazole,
- le 5-chloro 7-amino 1H-indazole,
- le 5-méthyl 7-amino 1H-indazole,
- le 6-méthyl 7-amino 1H-indazole,
- le 1-méthyl 4-amino 1H-indazole,
- le 1-méthyl 5-amino 1H-indazole,
- le 1-méthyl 6-amino 1H-indazole,
- le 1,3-diméthyl 6-amino 1H-indazole,
- le 1-méthyl 7-amino 1H-indazole,
- le 1,5-diméthyl 7-amino 1H-indazole,
- le 1-méthyl 3-bromo 7-(N-méthyl)-amino 1H-indazole,
- le 1-méthyl 3-chloro 7-(N-méthyl)-amino 1H-indazole,
- le 1-méthyl 5-chloro 7-amino 1H-indazole,
- le 1-méthyl 7-(N-méthyl)-amino 1H-indazole,
- le 1-méthyl 6-chloro 7-amino 1H-indazole,
- le 1-éthyl 7-amino 1H-indazole,
- le 2-méthyl 3-amino 5-trifluorométhyl 2H-indazole,
- le 2-méthyl 3- (N-méthyl) amino 2H-indazole,
- le 2-méthyl 4-amino 2H-indazole,
- le 2,6-diméthyl 4-amino 2H-indazole,
- le 2-méthyl 5-amino 2H-indazole,
- le 2,3-diméthyl 5-amino 2H-indazole,
- le 2-méthyl 6-amino 2H-indazole,
- le 2-méthyl 7-amino 2H-indazole,
- le 2-éthyl 5-amino 2H-indazole,
- le 2-éthyl 6-amino 2H-indazole,
- le 2-benzyl 4-amino 2H-indazole,
- le 2-(2'-N,N-dlméthyl)-aminoéthyl 6-amino 2H-indazole,
- le 2-(2'-N,N-diéthyl)-aminoéthyl 6-amino 2H-indazole,
- le 2-imidino 5-amino 2H-indazole,
- le 2-imidino 6-amino 2H-indazole,
- le 2-imidino 7-amino 2H-indazole,
et leurs sels d'addition avec un acide.

3. Composition selon l'une quelconque des revendications 1 ou 2, **caractérisée par le fait que** le ou les dérivés d'indazoles amines de formules (II) et/ou (III) et/ou le ou leurs sels d'addition avec un acide représentent de 0,0005 à 12 % en poids du poids total de la composition tinctoriale.

4. Composition selon la revendication 3, **caractérisée par le fait que** le ou les dérivés d'indazoles amines de formules (II) et/ou (III) et/ou le ou leurs sels d'addition avec un acide représentent de 0,005 à 6 % en poids du poids total de la composition tinctoriale.

5. Composition selon l'une quelconque des revendications précédentes, **caractérisée par le fait que** la ou les bases d'oxydation sont choisies parmi les paraphénylènediamines, les bis-phénylalkylènediamines, les paraaminophénols, les ortho-aminophénols, les bases hétérocycliques, et leurs sels d'addition avec un acide.

6. Composition selon l'une quelconque des revendications précédentes, **caractérisée par le fait que** les sels d'addition avec un acide sont choisis parmi les chlorhydrates, les bromhydrates, les sulfates, les tartrates, les lactates et les acétates.

7. Composition selon l'une quelconque des revendications précédentes, **caractérisée par le fait que** la ou les bases d'oxydation représentent de 0,0005 à 12 % en poids du poids total de la composition tinctoriale.

8. Composition selon la revendication 7, **caractérisée par le fait que** la ou les bases d'oxydation représentent de 0,005 à 6 % en poids du poids total de la composition tinctoriale.

9. Composition selon l'une quelconque des revendications précédentes, **caractérisée par le fait qu'**elle renferme en outre un ou plusieurs coupleurs additionnels différents des dérivés d'indazoles amines de formules (II) et (III) et/ou un ou plusieurs colorants directs.

10. Utilisation des dérivés d'indazoles amines de formules (II) ou (III) tels que définis à l'une quelconque des revendications 1 à 4 et 6, à titre de coupleurs dans des compositions pour la teinture d'oxydation des fibres kératiniques et en particulier des fibres kératiniques humaines telles que les cheveux, en association avec au moins une base d'oxydation.

11. Procédé de teinture d'oxydation des fibres kératiniques et en particulier des fibres kératiniques humaines telles que les cheveux, **caractérisé par le fait qu'**on applique sur ces fibres au moins une composition tinctoriale telle que définie à l'une quelconque des revendications 1 à 9, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

12. Procédé selon la revendication 11, **caractérisé par le fait que** l'agent oxydant est choisi parmi le peroxyde d'hydrogène, le peroxyde d'urée, les bromates de métaux alcalins, les persels tels que les perborates et persulfates, les peracides et les enzymes.

13. Dispositif à plusieurs compartiments, ou "kit" de teinture à plusieurs compartiments, dont un premier compartiment renferme une composition tinctoriale telle que définie à l'une quelconque des revendications 1 à 9 et un second compartiment renferme une composition oxydante.

## Claims

1. Composition for dyeing keratin fibres, and in particular human keratin fibres such as the hair, **characterized in that** it comprises, in a medium which is suitable for dyeing:
- as coupler, at least one amine indazole derivative chosen from
• the compounds of formula (II), and the addition salts thereof with an acid: 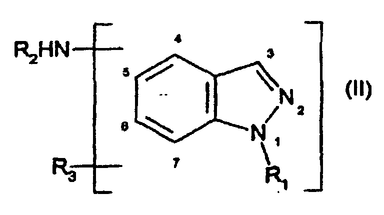
in which:
- R₁ denotes a hydrogen atom or a radical chosen from C₁-C₄ alkyl, C₁-C₄ hydroxyalkyl, C₁-C₄ aminoalkyl, mono(C₁-C₄)alkylamino(C₁-C₄)alkyl, di(C₁-C₄)alkylamino(C₁-C₄)alkyl, (C₁-C₄)alkoxycarbonyl, (C₁-C₄)alkoxycarbonyl(C₁-C₄)alkyl, C₁-C₄ acyl, (C₁-C₄)alkoxy(C₁-C₄)alkyl, cyano(C₁-C₄)alkyl, allyl and (C₁-C₄)alkylthiocarbonyl(C₁-C₄)alkyl radicals;
- R₂ denotes a hydrogen atom or a radical chosen from C₁-C₄ alkyl, C₁-C₄ hydroxyalkyl, C₁-C₄ aminoalkyl, mono(C₁-C₄)alkylamino(C₁-C₄)alkyl, di(C₁-C₄)alkylamino(C₁-C₄)alkyl, carboxy(C₁-C₄)alkyl, (C₁-C₄)alkoxy-(C₁-C₄)alkyl and ω-hydroxy(C₁-C₄)alkoxy(C₁-C₄)alkyl radicals;
- R₃ denotes a hydrogen atom, a bromine, chlorine or fluorine atom or a radical chosen from C₁-C₄ alkyl, C₁-C₄ hydroxyalkyl, C₁-C₄ aminoalkyl, C₁-C₄ alkoxy, (C₁-C₄)alkoxy(C₁-C₄)alkyl, mono(C₁-C₄)alkylamino-(C₁-C₄)alkyl, di(C₁-C₄)alkylamino(C₁-C₄)alkyl, C₁-C₄ perfluoroalkyl, cyano(C₁-C₄)alkyl and carboxy(C₁-C₄)-alkyl radicals;
it being understood that:
- when R₁ and R₂ simultaneously represent a hydrogen atom, then R₃ is other than a C₁ alkoxy radical;
• and the compounds of formula (III), and the addition salts thereof with an acid: 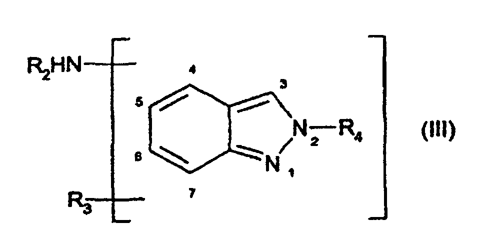
in which:
- R₄ denotes a radical chosen from C₁-C₄ alkyl, C₁-C₄ hydroxyalkyl, C₁-C₄ aminoalkyl, mono(C₁-C₄)alkylamino(C₁-C₄)alkyl, di(C₁-C₄)alkylamino(C₁-C₄)alkyl, (C₁-C₄)alkoxycarbonyl, C₁-C₄ acyl and C₇-C₉ alkylaryl radicals and an imidino radical (-C(NH₂)=NH) ;
- R₂ denotes a hydrogen atom or a radical chosen from C₁-C₄ alkyl, C₁-C₄ hydroxyalkyl, C₁-C₄ aminoalkyl, mono(C₁-C₄)alkylamino(C₁-C₄)alkyl, di(C₁-C₄)alkylamino(C₁-C₄)alkyl, carboxy(C₁-C₄)alkyl, (C₁-C₄)alkoxy(C₁-C₄)alkyl and ω-hydroxy(C₁-C₄)alkoxy(C₁-C₄)alkyl radicals;
- R₃ denotes a hydrogen atom, a bromine, chlorine or fluorine atom or a radical chosen from C₁-C₄ alkyl, C₁-C₄ hydroxyalkyl, C₁-C₄ aminoalkyl, C₁-C₄ alkoxy, (C₁-C₄)alkoxy(C₁-C₄)alkyl, mono(C₁-C₄)alkylamino-(C₁-C₄)alkyl, di(C₁-C₄)alkylamino(C₁-C₄)alkyl, C₁-C₄ perfluoroalkyl, cyano(C₁-C₄)alkyl and carboxy(C₁-C₄)alkyl radicals, or an amino group;
- and at least one oxidation base.

2. Composition according to Claim 1, **characterized in that** the indazole amine derivatives of formula (II) or (III) are chosen from:
- 4-amino-1H-indazole,
- 5-amino-1H-indazole,
- 6-amino-1H-indazole,
- 7-amino-1H-indazole,
- 4-(2'-aminoethyl)amino-1H-indazole,
- 5-(2'-aminoethyl)amino-1H-indazole,
- 4-amino-6-methyl-1H-indazole,
- 3-chloro-4-amino-1H-indazole,
- 4-amino-7-methyl-1H-indazole,
- 4-amino-5-methyl-1H-indazole,
- 4-(3'-aminopropyl)amino-1H-indazole,
- 3-fluoro-5-amino-1H-indazole,
- 4-methyl-5-amino-1H-indazole,
- 3-methyl-5-amino-1H-indazole,
- 3-[(2'-N,N-diethyl)aminopropyl]amino-5-amino-1H-indazole,
- 5-(2'-aminoethyl)amino-lH-indazole,
- 5-amino-6-methyl-1H-indazole,
- 3-trifluoromethyl-5-amino-1H-indazole,
- (5-amino-1H-indazol-3-yl)acetic acid,
- 3-chloro-5-amino-1H-indazole,
- 6-amino-7-chloro-1H-indazole,
- 5-amino-6-chloro-1H-indazole,
- 6-amino-7-bromo-1H-indazole,
- ethyl (6-amino-3-chloro-1H-indazol-1-yl)acetate,
- 1-methoxymethyl-6-amino-1H-indazole,
- 1-allyl-6-amino-1H-indazole,
- 6-amino-7-chloro-1H-indazole,
- (1H-indazol-6-yl-amino)acetic acid,
- 6-N-(2'-hydroxyethyl)amino-1H-indazole,
- 6-(N-propyl)amino-1H-indazole,
- 1-(N',N'-diethylamino)ethyl-6-amino-lH-indazole,
- 5-chloro-6-amino-1H-indazole,
- 1-(2'-cyano)ethyl-6-amino-1H-indazole,
- 3-(6-amino-1H-indazol-1-yl)thiopropionamide,
- 6-(N-ethyl)amino-lH-indazole,
- 3-methyl-6-amino-1H-indazole,
- 5-methyl-6-amino-1H-indazole,
- 3-bromo-6-amino-1H-indazole,
- 3-chloro-6-amino-1H-indazole,
- 6-amino-7-methyl-1H-indazole,
- 4-chloro-6-amino-1H-indazole,
- 5-methyl-7-(2'-methoxyethyl)amino-1H-indazole,
- 5-methyl-7-(2'-hydroxyethyl)amino-1H-indazole,
- 6-methyl-7-[2'-(2"-hydroxy)ethoxyethyl]amino-1H-indazole,
- 4-(2'-N,N-dipropyl)ethyl-7-amino-1H-indazole,
- 4-(2'-amino)ethyl-7-amino-1H-indazole,
- 4-cyanomethyl-7-amino-1H-indazole,
- 2-(2'-N,N-dimethyl)ethyl-7-amino-1H-indazole,
- 3-methyl-7-amino-1H-indazole,
- 4-chloro-7-amino-1H-indazole,
- 7-[N-(2'-amino)ethyl]amino-1H-indazole,
- 4-chloro-5-methyl-7-amino-1H-indazole,
- 5-chloro-7-amino-1H-indazole,
- 5-methyl-7-amino-1H-indazole,
- 6-methyl-7-amino-1H-indazole,
- 1-methyl-4-amino-1H-indazole,
- 1-methyl-5-amino-1H-indazole,
- 1-methyl-6-amino-1H-indazole,
- 1,3-dimethyl-6-amino-1H-indazole,
- 1-methyl-7-amino-1H-indazole,
- 1,5-dimethyl-7-amino-1H-indazole,
- 1-methyl-3-bromo-7-(N-methyl)amino-1H-indazole,
- 1-methyl-3-chloro-7-(N-methyl)amino-1H-indazole,
- 1-methyl-5-chloro-7-amino-1H-indazole,
- 1-methyl-7-(N-methyl)amino-lH-indazole,
- 1-methyl-6-chloro-7-amino-1H-indazole,
- 1-ethyl-7-amino-1H-indazole,
- 2-methyl-3-amino-5-trifluoromethyl-2H-indazole,
- 2-methyl-3-(N-methyl)amino-2H-indazole,
- 2-methyl-4-amino-2H-indazole,
- 2,6-dimethyl-4-amino-2H-indazole,
- 2-methyl-5-amino-2H-indazole,
- 2,3-dimethyl-5-amino-2H-indazole,
- 2-methyl-6-amino-2H-indazole,
- 2-methyl-7-amino-2H-indazole,
- 2-ethyl-5-amino-2H-indazole,
- 2-ethyl-6-amino-2H-indazole,
- 2-benzyl-4-amino-2H-indazole,
- 2-(2'-N,N-dimethyl)aminoethyl-6-amino-2H-indazole,
- 2-(2'-N,N-diethyl)aminoethyl-6-amino-2H-indazole,
- 2-imidino-5-amino-2H-indazole,
- 2-imidino-6-amino-2H-indazole,
- 2-imidino-7-amino-2H-indazole,
and the addition salts thereof with an acid.

3. Composition according to either of Claims 1 and 2, **characterized in that** the amine indazole derivative(s) of formulae (II) and/or (III) and/or the addition salt(s) thereof with an acid represent(s) from 0.0005% to 12% by weight relative to the total weight of the dye composition.

4. Composition according to Claim 3, **characterized in that** the amine indazole derivative(s) of formulae (II) and/or (III) and/or the addition salt(s) thereof with an acid represent(s) from 0.005% to 6% by weight relative to the total weight of the dye composition.

5. Composition according to any one of the preceding claims, **characterized in that** the oxidation base(s) is (are) chosen from para-phenylenediamines, bis(phenyl)alkylenediamines, para-aminophenols, ortho-aminophenols and heterocyclic bases, and the addition salts thereof with an acid.

6. Composition according to any one of the preceding claims, **characterized in that** the addition salts with an acid are chosen from the hydrochlorides, hydrobromides, sulphates, tartrates, lactates and acetates.

7. Composition according to any one of the preceding claims, **characterized in that** the oxidation base(s) represent(s) from 0.0005% to 12% by weight relative to the total weight of the dye composition.

8. Composition according to Claim 7, **characterized in that** the oxidation base(s) represent(s) from 0.005% to 6% by weight relative to the total weight of the dye composition.

9. Composition according to any one of the preceding claims, **characterized in that** it also contains one or more additional couplers other than the amine indazole derivatives of formulae (II) and (III) and/or one or more direct dyes.

10. Use of the indazole amine derivatives of formulae (II) or (III) as defined in any one of Claims 1 to 4 and 6, as couplers in compositions for the oxidation dyeing of keratin fibres, and in particular of human keratin fibres such as the hair, in combination with at least one oxidation base.

11. Process for the oxidation dyeing of keratin fibres, and in particular of human keratin fibres such as the hair, **characterized in that** at least one dye composition as defined in any one of Claims 1 to 9 is applied to these fibres, the colour being developed at acidic, neutral or alkaline pH using an oxidizing agent which is added to the dye composition just at the time of use, or which is present in an oxidizing composition that is applied simultaneously or sequentially in a separate manner.

12. Process according to Claim 11, **characterized in that** the oxidizing agent is chosen from hydrogen peroxide, urea peroxide, alkali metal bromates, persalts such as perborates and persulphates, peracids and enzymes.

13. Multicompartment device or multicompartment dyeing "kit", a first compartment of which contains a dye composition as defined in any one of Claims 1 to 9, and a second compartment of which contains an oxidizing composition.

## Patentansprüche

1. Zusammensetzung zum Färben von Keratinfasern und insbesondere menschlichen Keratirrfasern, wie dem Haar, **dadurch gekennzeichnet, daß** sie in einem zum Färben geeigneten Medium enthält:
- als Kuppler mindestens ein Indazolamin, das ausgewählt ist unter:
■ Verbindungen der folgenden Formel (II) und den Additionssalzen dieser Verbindungen mit einer Säure: 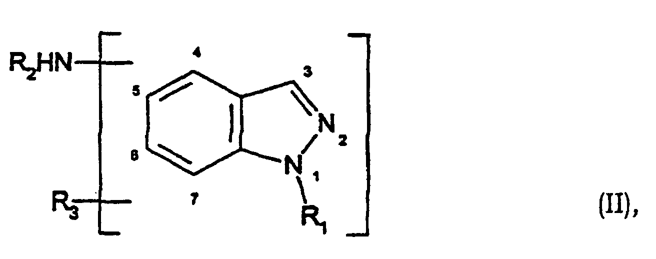 worin bedeuten:
- R₁ Wasserstoff oder eine Gruppe, die ausgewählt ist unter: C₁₋₄-Alkyl, C₁₋₄-Hydroxyalkyl, C₁₋₄-Aminoalkyl, C₁₋₄-Monoalkyl-C₁₋₄-aminoalkyl, C₁₋₄-Dialkyl-C₁₋₄-aminoalkyl, C₁₋₄-Alkoxycarbonyl, C₁₋₄-Alkoxy-C₁₋₄-carbonylalkyl, C₁₋₄-Acyl, C₁₋₄-Alkoxy-C₁₋₄-alkyl, C₁₋₄-Cyanoalkyl, Allyl und C₁₋₄-Alkyl-C₁₋₄-thiocarbonylalkyl;
- R₂ Wasserstoff oder eine Gruppe, die ausgewählt ist unter: C₁₋₄-Alkyl, C₁₋₄-Hydroxyalkyl, C₁₋₄-Aminoalkyl, C₁₋₄-Monoalkyl-C₁₋₄-aminoalkyl, C₁₋₄-Dialkyl-C₁₋₄-aminoalkyl, C₁₋₄-Carboxyalkyl, C₁₋₄-Alkoxy-C₁₋₄-alkyl und C₁₋₄-ω-Hydroxyalkoxy-C₁₋₄-alkyl;
- R₃ Wasserstoff, Brom, Chlor, Fluor oder eine Gruppe, die ausgewählt ist unter: C₁₋₄-Alkyl, C₁₋₄-Hydroryalkyl, C₁₋₄-Aminoalkyl, C₁₋₄-Alkoxy, C₁₋₄-Alkoxy-C₁₋₄-alkyl, C₁₋₄-Monoalkyl-C₁₋₄-aminoalkyl, C₁₋₄-Dlalkyl-C₁₋₄-aminoalkyl, C₁₋₄-Perfluoralkyl, C₁₋₄-Cyanoalkyl oder C₁₋₄-Carboxyalkyl; mit der Maßgabe, daß R₃ von C₁-Alkoxy verschieden ist, wenn R₁ und R₂ gleichzeitig Wasserstoff bedeuten;
■ Verbindungen der folgenden Formel (III) und den Additionssalzen dieser Verbindungen mit einer Säure: 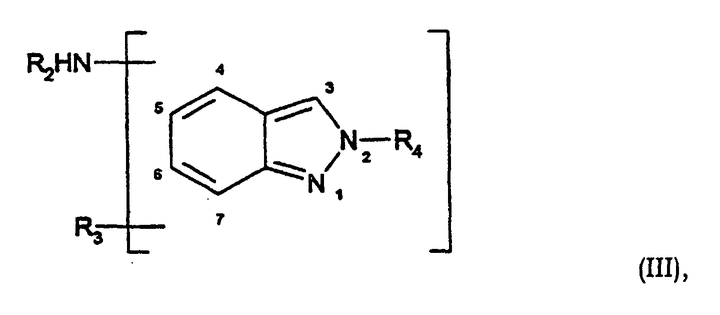 worin bedeuten:
- R₄ eine Gruppe, die ausgewählt ist unter: C₁₋₄-Alkyl, C₁₋₄-Hydroxyalkyl, C₁₋₄-Aminoalkyl, C₁₋₄-Monoalkyl-C₁₋₄-aminoalkyl, C₁₋₄-Dialkyl-C₁₋₄-aminoalkyl, C₁₋₄-Alkoxycarbonyl, C₁₋₄-Acyl, C₇₋₉-Alkylaryl oder Imidino (-C(NH₂)=NH);
- R₂ Wasserstoff oder eine Gruppe, die ausgewählt ist unter: C₁₋₄-Alkyl, C₁₋₄-Hydroxyalkyl, C₁₋₄-Aminoalkyl, C₁₋₄-Monoalkyl-C₁₋₄-aminoalkyl, C₁₋₄-Dialkyl-C₁₋₄-aminoalkyl, C₁₋₄-Carboxyalkyl, C₁₋₄-Alkoxy-C₁₋₄-alkyl und C₁₋₄-ω-Hydroxyalkoxy-C₁₋₄-alkyl;
- R₃ Wasserstoff, Brom, Chlor, Fluor oder eine Gruppe, die ausgewählt ist unter: C₁₋₄-Alkyl, C₁₋₄-Hydroxyalkyl, C₁₋₄-Aminoalkyl, C₁₋₄-Alkoxy, C₁₋₄-Alkoxy-C₁₋₄-alkyl, C₁₋₄-Monoalkyl-C₁₋₄-aminoalkyl, C₁₋₄-Dialkyl-C₁₋₄-aminoalkyl, C₁₋₄-Perfluoralkyl, C₁₋₄-Cyanoalkyl, C₁₋₄-Carboxyalkyl oder Amino;
und
- mindestens eine Oxidationsbase.

2. Zusammensetzung nach Anspruch 1, **dadurch gekennzeichnet, daß** die Indazolamine der Formeln (I) und (II) ausgewählt sind unter:
- 4-Amino-1H-indazol,
- 5-Amino-1H-indazol,
- 6-Amino-1H-indazol,
- 7-Amino-1H-indazol,
- 4-(2'-Amino-ethyl)-amino-1H-indazol,
- 5-(2'-Amino-ethyl)-amino-1H-indazol,
- 4-Amino-6-methyl-1H-indazol,
- 3-Chlor-4-amino-1H-indazol,
- 4-Amino-7-methyl-1H-indazol,
- 4-Amino-5-methyl-1H-indazol,
- 4-(3'-Amino-propyl)-amino-1H-indazol,
- 3-Fluor-5-amino-1H-indazol,
- 4-Methyl-5-amino-1H-indazol,
- 3-Methyl-5-amino-1H-indazol,
- 3-[(2'-N,N-Diethyl)-aminopropyl]-amino-5-amino-1H-indazol,
- 5-(2'-Amino-ethyl)-amino-1H-indazol,
- 5-Amino-6-methyl-1H-indazol,
- 3-Trifluormethyl-5-amino-1H-indazol,
- (5-Amino-1H-indazol-3-yl)-essigsäure,
- 3-Chlor-5-amino-1H-indazol,
- 6-Amino-7-chlor-1H-indazol,
- 5-Amino-6-chlor-1H-indazol,
- 6-Amino-7-brom-1H-indazol,
- (6-Amino-3-chlor-1H-indazol-1-yl)-essigsäureethylester,
- 1-Methoxymethyl-6-amino-1H-indazol,
- 1-Allyl-6-amino-1H-indazol,
- 6-Amino-7-chlor-1H-indazol,
- (1H-Indazol-6-yl-amino)-essigsäure,
- 6-N-(2'-Hydroxyethyl)-amino-1H-indazol,
- 6-(N-Propyl)-amino-1H-indazol,
- 1-(N',N'-Diethylamino)-ethyl-6-amino-1H-indazol,
- 3-Chlor-6-amino-1H-indazol,
- 1-(2'-Cyano)-ethyl-6-amino-1H-indazol,
- 3-(6-Amino-1H-indazol-1-yl)-thiopropionamid,
- 6-(N-Ethyl)-amino-1H-indazol,
- 3-Methyl-6-amino-1H-indazol,
- 5-Methyl-6-amino-1H-indazol,
- 3-Brom-6-amino-1H-indazol,
- 3-Chlor-6-amino-1H-indazol,
- 6-Amino-7-methyl-1H-indazol,
- 4-Chlor-6-amino-1H-indazol,
- 5-Methyl-7-(2'-methoxyethyl)-amino-1H-indazol,
- 5-Methyl-7-(2'-hydroxyethyl)-amino-1H-indazol,
- 6-Methyl-7-[2'-(2"-hydroxy)ethoxy-ethyl]-amino-1H-indazol,
- 4-(2'-N,N-Dipropyl)-ethyl-7-amino-1H-indazol,
- 4-(2'-Amino)-ethyl-7-amino-1H-indazol,
- 4-Cyanomethyl-7-amino-1H-indazol,
- 2-(2'-N,N-Dimethyl)-ethyl-7-amino-1H-indazol,
- 3-Methyl-7-amino-1H-indazol,
- 4-Chlor-7-amino-1H-indazol,
- 7-[N-(2'-Amino)-ethyl]-amino-1H-indazol,
- 4-Chlor-5-methyl-7-amino-1H-indazol,
- 5-Chlor-7-amino-1H-indazol,
- 5-Methyl-7-amino-1H-indazol,
- 6-Methyl-7-amino-1H-indazol,
- 1-Methyl-4-amino-1H-indazol,
- 1-Methyl-5-amino-1H-indazol,
- 1-Methyl-6-amino-1H-indazol,
- 1,3-Dimethyl-6-amino-1H-indazol,
- 1-Methyl-7-amino-1H-indazol,
- 1,5-Dimethyl-7-amino-1H-indazol,
- 1-Methyl-3-brom-7-(N-methyl)-amino-1H-indazol,
- 1-Methyl-3-Chlor-7-(N-methyl)-amino-1H-indazol,
- 1-Methyl-5-chlor-7-amino-1H-indazol,
- 1-Methyl-7-(N-methyl)-amino-1H-indazol,
- 1-Methyl-6-chlor-7-amino-1H-indazol,
- 1-Ethyl-7-amino-1H-indazol,
- 2-Methyl-3-amino-5-trifluormethyl-2H-indazol,
- 2-Methyl-3-(N-methyl)-amino-2H-indazol,
- 2-Methyl-4-amino-2H-indazol,
- 2,6-Dimethyl-4-amino-2H-indazol,
- 2-Methyl-5-amino-2H-indazol,
- 2,3-Dimethyl-5-amino-2H-indazol,
- 2-Methyl-6-amino-2H-indazol,
- 2-Methyl-7-amino-2H-indazol,
- 2-Ethyl-5-amino-2H-indazol,
- 2-Ethyl-6-amino-2H-indazol,
- 2-Benzyl-4-amino-2H-indazol,
- 2-(2'-N,N-Dimethyl)-aminoethyl-6-amino-2H-indazol,
- 2-(2'-N,N-Diethyl)-aminoethyl-6-amino-2H-indazol,
- 2-Imidino-5-amino-2H-indazol,
- 2-Imidino-6-amino-2H-indazol,
- 2-Imidino-7-amino-2H-indazol, und
- deren Additionsalze mit einer Säure.

3. Zusammensetzung nach einem der Ansprüche 1 oder 2, **dadurch gekennzeichnet, daß** das Indazolamin oder die Indazolamine der Formeln (I) und/oder (II) und/oder deren Additionssalz(e) mit einer Säure 0,0005 bis 12 Gew.-% des Gesamtgewichts der Farbmittelzusammensetzung ausmachen.

4. Zusammensetzung nach Anspruch 3, **dadurch gekennzeichnet, daß** das Indazolamin oder die Indazolamine der Formeln (I) und/oder (II) und/oder deren Additionssalz(e) mit einer Säure 0,005 bis 6 Gew.-% des Gesamtgewichts der Farbmittelzusammensetzung ausmachen.

5. Zusammensetzung nach einem der vorhergehenden Ansprüche, **dadurch gekennzeichnet, daß** die Oxidationsbase(n) unter den p-Phenylendiaminen, Bisphenylalkylendiaminen, p-Aminophenolen, o-Aminophenolen, heterocyclischen Basen und deren Additionssalzen mit einer Säure ausgewählt sind.

6. Zusammensetzung nach einem der vorhergehenden Ansprüche, **dadurch gekennzeichnet, daß** die Additionssalze mit einer Säure unter den Hydrochloriden, Hydrobromiden, Sulfaten, Tartraten, Lactaten und Acetaten ausgewählt sind.

7. Zusammensetzung nach einem der vorhergehenden Ansprüche, **dadurch gekennzeichnet, daß** die Oxidationsbase(n) etwa 0,0005 bis 12 Gew.-% des Gesamtgewichts der Farbmittelzusammensetzung ausmachen.

8. Zusammensetzung nach Anspruch 7, **dadurch gekennzeichnet, daß** die Oxidationsbase(n) 0,005 bis 6 Gew.-% des Gesamtgewichts der Farbmittelzusammensetzung ausmachen.

9. Zusammensetzung nach einem der vorhergehenden Ansprüche, **dadurch gekennzeichnet, daß** sie ferner einen oder mehrere zusätzliche Kuppler, die von den Indazolaminderivaten der Formeln (I) und (II) verschieden sind, und/oder einen oder mehrere Direktfarbstoffe enthält.

10. Verwendung von Indazolaminderivaten der Formeln (I) oder (II) nach einem der Ansprüche 1 bis 4 und 6 als Kuppler in Zusammensetzungen zum oxidativen Färben von Keratinfasern und insbesondere menschlichen Keratinfasern, wie dem Haar, in Kombination mit mindestens einer Oxidationsbase.

11. Verfahren zum oxidativen Färben von Keratinfasern und insbesondere menschlichen Keratinfasern, wie dem Haar, **dadurch gekennzeichnet, daß** auf die Fasern mindestens eine Farbmittelzusammen-Setzung nach einem der Ansprüche 1 bis 9 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

12. Verfahren nach Anspruch 11, **dadurch gekennzeichnet, daß** das Oxidationsmittel unter Wasserstoffperoxid, Harnstoffperoxid, Alkalimetallbromaten, Salzen von Persäuren, wie Perboraten und Persulfaten, und Enzymen ausgewählt ist.

13. Vorrichtung mit mehreren Abteilungen oder "Kit" zum Färben mit mehreren Abteilungen, wobei eine erste Abteilung eine Farbmittelzusammensetzung nach einem der Ansprüche 1 bis 9 und eine zweite Abteilung eine oxidierende Zusammensetzung enthält.
